# EUROPEAN PATENT SPECIFICATION

(11) **EP 1 750 573 B1**
(45) Date of publication and mention of the grant of the patent: **27.04.2011**
(21) Application number: 05733993.9
(22) Date of filing: 09.05.2005
(51) Int. Cl.: H04W 4/02, G06F 19/00

(54) **Method and apparatuses for location and access right dependent access control**
Verfahren und Vorrichtungen fuer eine Orts- und zugriffsrechtabhaengige Zugangskontrolle
Procédé et dispositifs pour une commande d'acces en fonction du lieu et des droits d'access

(30) Priority: 13.05.2004 EP 04102093
(43) Date of publication of application: 14.02.2007
(73) Proprietor: Philips Intellectual Property & Standards GmbH, 20099 Hamburg (DE); Koninklijke Philips Electronics N.V., 5621 BA Eindhoven (NL)
(72) Inventor: KAHLERT, Joachim, 52066 Aachen (DE); BAUMEISTER, Markus, 52066 Aachen (DE); WISCHHUSEN, Olaf, 52066 Aachen (DE)
(74) Representative: Volmer, Georg
(86) International application number: PCT/IB2005/051504
(87) International publication number: WO 2005/110208

(56) References cited:
- WO-A-00/62574
- DE-A1- 3 036 217
- US-A1- 2002 164 997

## Description

The present invention relates to the field of wireless control of resources of a network and in particular to wireless control of medical devices.

A network providing various resources to a plurality of users is typically accomplished with an access protection mechanism. By means of such an access protection mechanism, access to the resources of the network may be selectively granted or denied for a distinct user or a distinct group of users. By means of the access protection mechanism a distinct user can be authorized to make use of a particular network resource of e.g. a computer network, such as reading a particular database entry, writing a database entry, executing a particular software application, configuring a software application or a software application driven apparatus connected to the computer network.

Authorization of a user is typically performed by means of a unique user ID and a corresponding password. Before making use of components of a computer network, the user has to enter his or her user ID in combination with the corresponding password in order to legitimate with the computer network. Depending on a predefined access scheme, the user then has full or limited access to various resources of the computer network or access to various network resources is denied.

In the framework of wireless network technology, a user typically accesses network resources by making use of a mobile device. Here, the access to the network resources is based on a wireless technology allowing the user to arbitrarily move within a certain radius. Since the user and his mobile device are non stationary, a location information being indicative of the location of the mobile device could be effectively exploited by an access protection mechanism.

The US patent application 2002/0164997 A1 discloses a method for controlling wireless communication access. This method includes defining a zone with at least one first computing device and then selectively permitting wireless communication access for a second computing device to the first computing device based on a position of the second computing device relative to the zone. The first computing device acts as a gateway for controlled wireless communication access by second computing device to first computing device or for example a computer system with software application. Based upon the absolute or relative position of second computing device, relative to an access zone adjacent to the position of at least one first computing device, second computing device will be selectively denied or permitted access to first computing device.

Even though US 2002/0164997 A1 discloses a position dependent control of wireless communication access, it does not account for a multi-user environment with a plurality of users having different predefined levels of authorization. The document only focuses on an absolute and/or relative position of first and second computing devices irrespectively of any legitimation of a user making use of the first or second computing devices.

WO00/62574 A discloses a method of generating control signals based on the location of radio terminals. The method includes the steps of determining the location of a first and a second mobile radio terminal, comparing the locations of the terminals, and generating a control signal based upon the comparison.

DE3036217A1 discloses a medical device that is wireless controlled by a control unit. The control unit can only be operated at specific locations.

US2002/0164997 discloses a method and system for controlling selective wireless communication access. Wireless communication is controlled by defining an access zone with at least one first computing device and then selectively permitting wireless communication access for a second computing device to the first computing device based on a position of the second computing device relative to the access zone.

Location based access to data or applications provided by a computer system is particularly advantageous in the medical environment, such as e.g. in a hospital. When for example a doctor making use of a mobile wireless computing device comes in close proximity to a patient, the doctor typically desires to access all patient relevant records that are stored in a computer database.

The present invention therefore aims to provide an improved method of wireless control of a medical device by making use of a mobile device.

The present invention provides a method of wireless entry of a control command from a first mobile device into a medical device according to independent claim 1. Corresponding independent apparatus claims 3 and 4 concern a mobile device and a network element, respectively. The method therefore comprises the steps of determining a first location of the first mobile device, evaluating an access condition for the wireless entry from the first mobile device and enabling the wireless entry of the control command if the access condition is fulfilled. The access condition depends on the distance between the first location and the location of the medical device. For example, the access condition may only evaluate to true and hence allow to enable the wireless entry of the control command if the distance between the first location and the location of the medical device is below a predefined threshold. Since the mobile device serves as a kind of remote control for the medical device and being adapted for receiving control commands from a user, a remote controlling functionality of the first mobile device can be implemented as location dependent with respect to the location of the medical device.

In this way, the invention provides an effective means for allowing of access to a medical device only when a user is in close proximity to the medical device. In such a case it can be assumed that the user not only controls but also surveys the functionality of the medical device. Hence, a mistaken usage of the medical device in the absence of a user or an operator can be effectively prevented.

The inventive method makes use of determining the first location of the first mobile device and therefore also precisely locates the position of a user making use of the mobile device. Since the wireless entry of the control command into the medical device is only enabled if the access condition is fulfilled, the impact of the first mobile device on the medical device can be completely specified by the access condition. The access condition may provide a plurality of various control scenarios.

In a basic embodiment of the invention, the access condition makes use of only two parameters: the location of the first mobile device and the location of the medical device. In this embodiment, it is assumed that the medical device is stationary, hence the location of the medical device remains fixed. In contrast, the first location of the first mobile device may arbitrarily change. In a first example the access condition may specify an access zone with respect to the location of the medical device. In this case the access condition would be fulfilled when the distance between the first location and the location of the medical device is within and/or above predefined thresholds. For example a doctor making use of a medical device by means of a mobile device may be enabled to enter a command into the medical device only when he is in close proximity to the medical device. Alternatively, the doctor may only be enabled to enter the command when he is located within a predefined margin from the medical device or when the doctor is even separated at least a predefined distance from the medical device. This latter alternative is particularly reasonable for medical devices emitting high energetic radiation, e.g. X-ray imaging apparatuses.

Moreover, the access condition may also be specific for each one of a plurality of medical devices. Alternatively, there may exist a particular access condition for each medical device specifying location dependent access to the medical device.
Furthermore, the access condition may be specific for the first mobile device. In this way a mobile device may be enabled for wireless entry a control command into a first medical device while simultaneously being disabled to entry a control command into a second medical device. Hence, by means of the access condition, the first mobile device can be selectively enabled to enter a control command into a distinct medical device or into a particular group of medical devices.

According to a further preferred embodiment of the invention, the access condition further specifies various levels of access. Levels of access are for example access for reading a database entry, access for writing a database entry, access for executing a software application and/or access for modifying the configuration of the medical device. Therefore, by means of the access condition the entry of a variety of control commands can be enabled or disabled depending on the first location with respect to the location of the medical device. For example, a doctor may be enabled to remotely switch on a medical device but being simultaneously disabled to invoke any further function of the medical device.

According to a further preferred embodiment of the invention, the method of wireless entry of a control command from a first mobile device further comprises determining of a first user ID of a first user entering the control command into the first mobile device. In this embodiment evaluation of the access condition further depends on this first user ID. In this way, the inventive method further supports a multi-user environment and effectively allows for a user specific enabling or disabling of wireless entry of the control command into the medical device.

By making use of a user ID, the method effectively accounts for various levels of authorization of different users of the first mobile device. In order to provide user specific authorization, each user of a mobile device has to pass a login procedure by entering his user ID in combination with a valid password. Once the user has legitimized, his location is tracked by determining the first location of the first mobile device. The access condition may then further be specific for enabling wireless entry of a control command into the medical device with respect to the location of the first mobile device, the location of the medical device and the user ID of the user of the first mobile device. In this way, a doctor may for example be enabled to make use of a medical device within a relatively large distance from the medical device whereas a lower skilled nurse might only be enabled to make use of the same medical device when she is in close proximity to the medical device.

According to a further preferred embodiment of the invention, the method of wireless entry of the control command from the first mobile device further comprises determining of a second user ID of a second user of a second mobile device and determining a second location of the second mobile device. In this embodiment the evaluation of the access condition further depends on the second user ID and the distance between the first location and the second location. Hence, the access condition can be evaluated with respect to five parameters: location of the medical device, location of the first mobile device, location of the second mobile device, user ID of the first user and user ID of the second user, whereby the first and the second user using the first and the second mobile device, respectively.

In this way, the wireless entry of the control command from the first mobile device may be enabled when a second user making use of a second mobile device is located nearby the first user that makes use of the first mobile device. Such a functionality is for example advantageous when due to safety aspects a distinct medical device has to be operated by at least two users simultaneously. Moreover, enabling of the wireless entry may depend on the relative distance between the first and the second user. This is for example reasonable when the first user is instructed or supervised by the second user.

According to a further preferred embodiment of the invention, the method of wireless entry of a control command further comprises inheriting second access rights of the second user to first access rights of the first user if the distance between the first and the second location is within a predefined threshold. In this embodiment the evaluation of the access condition further depends on the first and second access rights. Here, access rights specify a predefined level of authorization of a user. Typically, a highly authorized user of a mobile device, such as a doctor, has high level access rights and a low authorized personnel like a nurse is associated with low level access rights. Since both a user with high level access rights and a user with low level access rights make independently use of a mobile device, the user with the low level access rights has only limited access to the functionality of a medical device compared to the user having high level access rights. Under predefined conditions, when for example the distance between the first location and the second location is below a predefined threshold, the high level access rights of e.g. a doctor can be transferred to the person featuring only low level access rights normally. Hence, high level access rights can be effectively inherited from a second user to a first user when the first and the second user are in close proximity. For example, the doctor may simply instruct a lower skilled personnel with a low level access right for making an entry into a patient database on the behalf of the doctor.

According to a further preferred embodiment of the invention, enabling of the wireless entry and evaluation of the access condition being repeatedly performed in order to respond to a modification of the distance between the first and the second location and/or a modification of the distance between the first location and the location of the medical device. By permanently determining the first and the second location of the mobile devices in combination with repeatedly enabling of the wireless entry and repeatedly evaluating of the access condition, the inventive method can dynamically respond to any changes referring to the first and second location. In this way, it can effectively be guaranteed that a user accomplished with low level access rights receives higher level access rights only as long as he moves in close proximity to a user having higher level access rights. For example, a nurse has a privileged access to a patient record only as long as she moves in close proximity to a doctor. As soon as the nurse moves away from the doctor, her access to the patient record is again restricted to reading of the patient record.

In another aspect, the invention provides a mobile device for wireless entry of a control command into a medical device. The mobile device comprises means for entering the control command into the mobile device, means for determining a first location of the mobile device, means for evaluating an access condition for the wireless entry of the control command and means for enabling the entry of the control command if the access condition is fulfilled. Thereby, the access condition depends on the first location and a location of the medical device. The inventive mobile device is adapted to wirelessly communicate with the medical device and/or with a network having access to the medical device.

The means for entering the control command into the mobile device are preferably adapted as a user interface providing information related to the medical device to the user. The user interface being further adapted to transmit control commands of the user either to the medical device or to the network. The means for determining a first location of the mobile device are for example implemented as a global positioning satellite (GPS) unit. Such a GPS unit may be implemented into the mobile device. Alternatively, the means for determining the first location of the mobile device may be implemented by means of triangulation making use of a plurality of receivers being adapted to receive a signal transmitted by the mobile device. In this case, the means for determining the first location of the mobile device only comprise a transmitter being adapted for transmitting a predefined sequence of electromagnetic signals. By receiving the transmitted sequence of electromagnetic signals by a plurality of corresponding receivers that are spatially separated, the location of the mobile device can be precisely determined. In this case determination of the location of the mobile device is preferably performed by some component of the network. The means for determining of the first location are then additionally adapted for receiving a location information from the corresponding network component.

The means for enabling the entry of the control command are adapted to wirelessly communicate with the medical device or to wirelessly communicate with the network in order to enter the control command into the medical device via the network.

In another aspect, the invention provides a network element for wireless entry of a control command into a medical device. The inventive network element comprises means for wireless receiving of the control command from a first mobile device, means for determining a first location of the first mobile device, means for evaluating an access condition for the wireless entry of the control command and means for enabling the entry of the control command if the access condition is fulfilled. Here, the access condition depends on the first location and a location of the medical device. In this embodiment, the means for determining the first location of the first mobile device are implemented into the network element. Furthermore, the first mobile device is typically accomplished with a transmitter that transmits a predefined sequence of electromagnetic signals that are detectable by a plurality of receivers being interconnected to the network element via the network.

According to a further preferred embodiment of the invention, the network element further comprises an access database that is adapted to store assignments between access conditions, user ID's and first locations. The access database is further adapted to provide an access condition of a user in response to receiving a first location and a first user ID.

According to a further preferred embodiment of the invention, the access database is further adapted to store assignments between access conditions, first user ID's, second user ID's and first locations as well as second locations. Hence, by means of the access database inheritance of access rights from a second user to a first user can be specified. Also, the access database may specify under which conditions an inheritance of access rights may be enabled.

In another aspect, the invention provides a method of wireless entry of a control command from a first mobile device into a resource of a network wherein a first user has a first user ID and wirelessly accesses the network by means of the first mobile device. The method of wireless entry of a control command into a resource of a network comprises the steps of determining a first location of the first mobile device, determining a second location of a second mobile device, determining a second user ID of a second user accessing the network by means of the second mobile device, determining an access right of the first user to the resource of the network and enabling the wireless entry of the control command if the access right is sufficient.

Here, the access right depends on the second user ID and further depends on the distance between the first location and the second location. In this way a first user having only low level access rights can obtain higher level access rights when being in close proximity to a second user that has high level access rights. Hence, access rights can be inherited between any two users of the network depending on their mutual distance. This feature is particularly advantageous for teaching, demonstrating and/or supervision purposes. For example, when a low level authorized person, e.g. a nurse is supervised by a high level authorized person, e.g. a doctor, the nurse obtains the doctor's access rights in order to perform some supervised actions while being instructed by the doctor.

According to a further preferred embodiment of the invention, enabling of the wireless entry and evaluation of the access condition being repeatedly performed in order to respond to a modification of the distance between the first and the second location. This means, that the location of the second and the first user are permanently checked. As soon as the distance between the first and the second user remarkably changes, the access rights of the first user are repeatedly determined. In case the mutual distance between the two users exceeds a predefined threshold, hence a supervision by the second user can no longer be guaranteed, the access status of the first user will return to the low level access rights. In this way the inventive method of wireless entry of a control command provides a dynamic and location dependent allocation of access rights.

Moreover, not only the mutual distance between the first user and the second user but also the distance to a e.g. medical device can be taken into account. For example, both the first and the second user may have access to a resource of the network, e.g. a medical device, when they are both in close proximity to the network resource.

According to a further preferred embodiment of the invention, access rights of a user refer to read and write access of a database and/or access to execution of particular software applications controlling operation of various devices connected to the network. For example in a medical care environment access rights to a database may refer to access rights of patient records and execution rights of a software application may refer to location dependent remote control of medical devices.

According to a further preferred embodiment of the invention, the method of wireless entry of a control command from a first mobile device into a resource of a network makes preferable use of an access database. The access database is adapted to store assignments between access rights, user ID's and first locations as well as second locations. By means of the access database inheritance of access rights from a second user ID to a first user ID can be precisely specified. Furthermore, the access database specifies threshold conditions for the mutual distance between the first user and the second user. By means of these threshold conditions, inheritance of access rights from the second user to the first user can be controlled with respect to a mutual distance between the first and the second user and/or with respect to a distance to a network resource.

In the following preferred embodiments of the invention will be described in greater detail by making reference to drawings in which:
Figure 1 shows a block diagram of a first mobile device being adapted for wireless entry of a control command into a medical device,
Figure 2 shows a block diagram of a first and a second mobile device for wireless entry of a control command into the medical device by making use of an access database,
Figure 3 is illustrative of a flow chart of the method of an inheritance of access rights,
Figure 4 shows a block diagram of a mobile device,
Figure 5 illustrates a flow chart of location dependent inheritance of access rights by making use of an access database query.

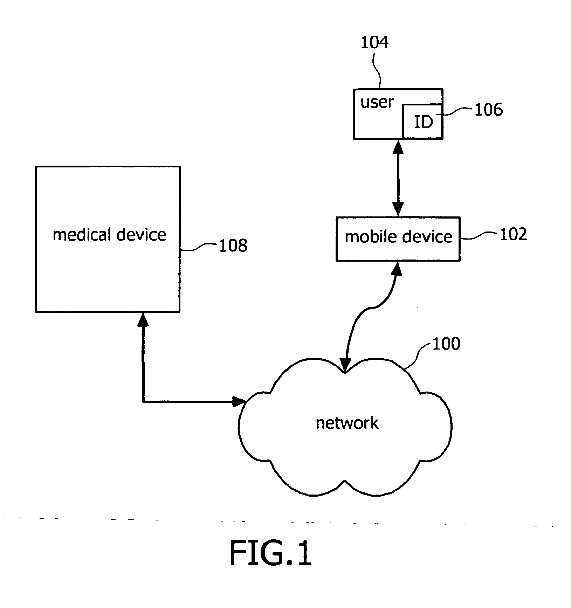

The block diagram of figure 1 shows a network 100, a mobile device 102, a user 104 as well as a medical device 108. The user 104 has a user ID 106 allowing for unique identification of the user 104. The user 104 makes use of the mobile device 102 that is adapted to wirelessly communicate with the network 100. Wireless communication is preferably based on infrared and/or radio frequency communication techniques making use of communication standards, such as Bluetooth, wireless LAN, IEEE 802.11, Ultra Wideband (UWB) or IrDA protocols.

The medical device 108 serves as an example of a network resource. Operation and functionalities of the medical device 108 and/or the network resource can in general be controlled by the mobile device 102 via the network 100. Communication between the network resource 108 and the network is preferably realized by wired communication since the medical device 108 is usually stationary. Alternatively, the network resource or the medical device 108 can also be implemented as a mobile medical device. In such a case communication between the network resource, e.g. the mobile medical device 108 and the network 100 can be also implemented as wireless communication based on the same technique as the wireless communication between the network 100 and the mobile device 102.

The user 104 identifies to the network 100 by means of his user ID 106 and a corresponding password. The user ID 106 serves to identify each user 104 of the network and to specify the access rights of each user 104 to the medical device 108 or to the network resource 108. Since various operations of the medical device 108 or the network resource 108 require different levels of authorization of a user 104, access to the operations of the medical device 108 is only granted to a user 104 having the appropriate access right. In the illustrated embodiment, the medical device 108 is only accessible via the network 100. Therefore, the network 100 controls the access of the user 104 to the medical device 108.

A control command entered by the user 104 into the mobile device 102 is wirelessly transferred to the network 100. Additionally, a location of the mobile device 102 is determined. Determination of the location can either by performed by means of a GPS module being implemented into the mobile device 102 or by means of a plurality of signal transmitters and receivers that are controlled by the network 100. In the latter case a location of the mobile device 102 can be precisely determined by making use of triangulation.

Having received the control command from the mobile device 102, the user ID 106 of the user 104 of the mobile device 102, and the location of the mobile device 102, an appropriate access condition for the network resource or medical device 108 can be evaluated by the network 100. The access condition specifies who can access which functionality of the network resource or the medical device 108 at which distance or location of the mobile device 102 with respect to the network resource or medical device 108. In case the medical device 108 is stationary, the network 100 preferably has location information of the medical device 108. In this way receiving location information of the mobile device 102 allows to determine a mutual distance between the mobile device 102 and the medical device 108.

Once the access condition has been determined by the network 100, the control command received from the mobile device 102 is enabled and the control command is executed by the network resource 108 or medical device 108. A control command may refer to a database command like read or write an entry of a patient record. It may also refer to an execution command or a distinct operation mode of a medical device 108. By means of the access condition for each medical device 108, various access conditions can be specified for each user 104 of a mobile device 102. If for example the medical device is an X-ray examination apparatus, the access condition may specify that operation of the medical device is only enabled when the mutual distance between the mobile device 102 and the medical device 108 is sufficiently large. In contrast, other medical devices may require the presence and close proximity of highly educated and authorized medical personnel. In such a case a wireless entry of the control command is only enabled when the distance between the mobile device 102 and the medical device 108 is sufficiently small.

The mobile device 102 is preferably implemented as a hand held computer, like a laptop computer, a personal digital assistance (PDA) or any other portable electronic device providing wireless communication with the network 100.

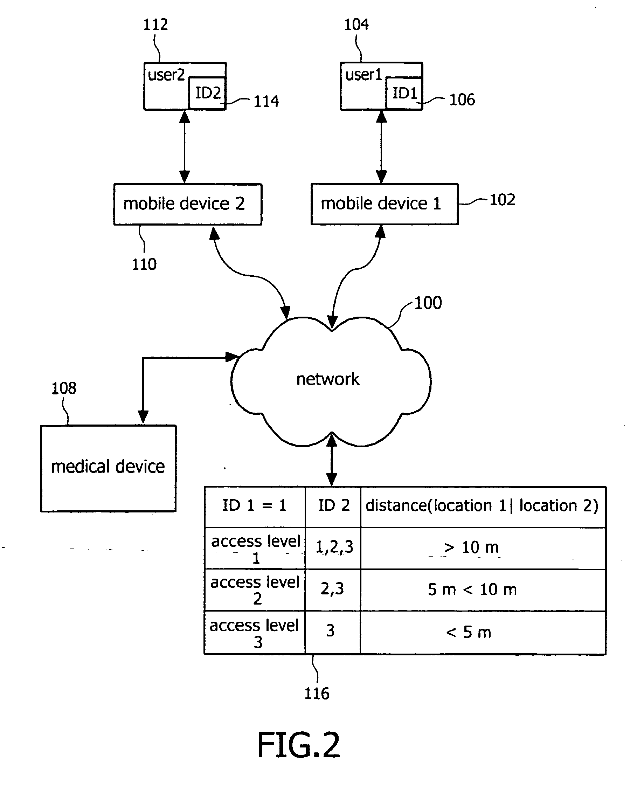

Figure 2 is illustrative of a block diagram of a configuration of two users making use of the network 100 for entering a control command into the network resource or medical device 108. Similar as in the embodiment of figure 1 the user 104 having a user ID 106 makes use of the mobile device 102 to access the network 100. In the same way user 112 having user ID 114 makes use of the mobile device 110 for accessing the same network 100. Also here, the network resource or medical device 108. is connected to the network 100. Hence, the functionality of the network resource or the medical device 108 can be controlled via the network 100 by means of fixed communication or even wireless communication techniques. Additionally, the table 116 is illustrative of one example of an access database providing a plurality of access conditions. The access database 116 is either a part of the network 100 or is implemented as a separate component that can be accessed by the network 100.

Wireless entry of control commands from either mobile device 102 or mobile device 110 is principally performed in the same way as described in figure 1. Since a first and a second user 104, 112 simultaneously communicate and access the network 100, the location of both mobile devices 102, 110 can be taken into account for evaluating an access condition for the wireless entry of the control command. The network 100 is typically provided with location information of both mobile devices 102, 110. Moreover, the network receives information of the identity of the users 104, 112.

Upon communication between mobile devices 102, 110 with the network 100 user ID's 106, 114 are transmitted to the network 100. In this way, the network 100 is able to uniquely identify the user 112 of the mobile device 110 and the user 104 of the mobile device 102. Each user ID 106, 114 allows to determine the authority and an access level of the corresponding user 104, 112.

After determination of the location of mobile device 110 and mobile device 102 as well as user ID 114 and user ID 106, an appropriate location based access condition can be evaluated. Table 116 gives an example of various access conditions. The access table 116 is specific for the access level of user 104 having user ID 106. The first column of access table 116 shows three different access levels 1, 2, 3 for the user 104 with user ID 106. In this case user ID 106 specifies a low level authorized user 104. Hence, the default access level of user 104 is equal to 1, specifying the lowest access level. The second column of access table 116 specifies access levels of the second user 112 with user ID 114. Finally, the third column specifies a range of the distance between the mobile device 102 and mobile device 110.

The second row of the access table 116 reflects an access condition for assigning access level 1 to user 104. Access level 1 is assigned to user 104 when user ID 114 is either of access level 1, 2 or 3 and when the distance between the location of the two mobile devices 110, 102 is larger than 10 meters.

The third row of the access table 116 is illustrative of an access condition for assigning access level 2 to user 104. Referring to the third row of access table 116, access level 2 is assigned to user 104 when access level of user ID 114 is either of access level 2 or 3 and when the distance between the location of mobile device 110 and mobile device 102 is within a range of 5 to 10 meters.

The fourth row of access table 116 finally specifies an access condition for assigning the highest access level 3 to user 104. Access level 3 is only assigned to user 104 when the user ID 114 of user 112 equals access level 3 and when the distance between the two corresponding mobile devices is beneath 5 meters.

In this way different access levels can be assigned to user 104 with respect to an access level of user 112 and a mutual distance between mobile devices 110, 102. In this embodiment access table 116 refers to a two dimensional matrix but can be in general expanded to a multi-dimensional matrix in order to control access rights of user 104 with respect to additional parameters, as e.g. relative position of mobile device 102 and/or mobile device 110 with respect to the location of the network resource or the medical device 108.

Preferably, evaluation of the access condition is performed dynamically. Modifications of the absolute or relative position of mobile devices 110, 102 are monitored and evaluation of the access condition is permanently updated. In case a particular access condition is no longer fulfilled due to a modification of the relative position of the mobile devices 110, 102, access to the network resource or the medical device 108 is disabled in response. In the illustrated embodiment the third column of the access table 116 specifies various thresholds for the mutual distance between the mobile devices 102, 110. When for example user 112 has the highest access level 3 and is in close proximity to user 104 with the lowest access level 1, user 104 may inherit access rights of user 112 when their corresponding mobile devices are closer than 5 meters.

As soon as user 104 and his mobile device 102 depart from user 112 and the mobile device 110 such that the mutual distance between the mobile devices exceeds 5 meters, the access condition specified by the fourth row of the table 116 is no longer valid. When the mutual distance between the two mobile devices 102, 110 becomes for example 7 meters, the access condition specified in row 3 of table 116 holds and consequently user 104 is assigned with access level 2. As soon as the two users and their mobile devices 102, 110 mutually approach, such that the relative distance between the mobile devices 102, 110 becomes less than 5 meters, access level 3 is reassigned to user 104.

In this embodiment the distance conditions as they are specified by the third column refer only to a mutual distance between mobile devices 102, 110. Hence the distance conditions specify a radius for the location of mobile device 110. More elaborate position conditions may also refer to an absolute position of mobile devices 110, 102 and may even take into account an orientation of mobile devices 110, 102. Moreover, a distance condition does not necessarily has to refer to a certain radius but can also specify a more complex area in which access to a network resource or medical device 108 is granted to the user 104.

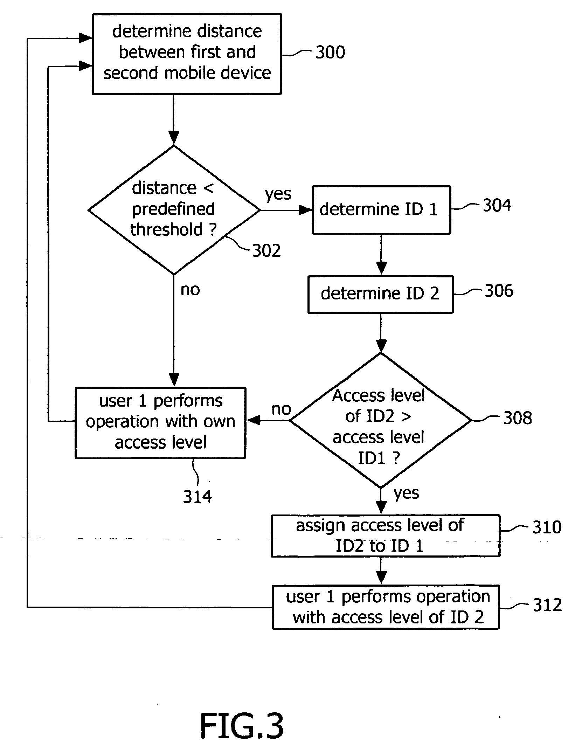

Figure 3 is illustrative of a flow chart for location dependent inheritance of access rights from a second user to a first user. In a first step 300 the distance between a first and a second mobile device is determined. In a following step 302, it is checked whether the determined distance is below a predefined threshold. If the distance is above the predefined threshold, i.e. the first and the second user that make use of the first and the second mobile device are separated by a rather large distance, the method continues with step 314.

In step 314 the first user performs an operation with his own, default access level. This means, that the first user enters a control command into his mobile device and that the mobile device wirelessly transfers the entered control command to a network resource or medical device only when the access level of the first user is sufficient for execution of the entered control command. Hence, depending on the default access level of the first user, a corresponding operation is performed. After performing the operation in step 314 the method returns to step 300 and repeatedly determines the distance between the first and the second mobile device.

When in step 302 it is determined that the mutual distance between the first and the second mobile device is below a predefined threshold the method continues with step 304. In step 304 a user ID of the user of the first mobile device is determined and in the following step 306 a corresponding user ID of a second user of the second mobile device is determined. In the successive step 308 the two access levels corresponding to the user ID of the first and the second user are compared. It is checked whether the second user ID specifies a higher level access than the first user ID.

When in step 308 it is determined that the second user does not have a higher level authorization than the first user, the method continues with step 314 in the same way as before. Only in case that access level of the second user is larger than the access level of the first user, the method continues with step 310, wherein access level of the second user is assigned to the first user. In this way a high level authority of the second user is inherited to the first user. In a subsequent step 312 the first user can then perform an operation by making use of the access level of the second user. Hence, higher level access is temporarily assigned to the first user due to the proximity of the two mobile devices.

Typically, the method illustrated in this flow chart is performed by making use of an access table 116. The access table specifies a predefined threshold for mutual distances between first and second mobile devices as well as additional specifications that are useful for inheritance of access rights. The access table may for example specify that for a given operation inheritance is generally prohibited, i.e. only a high level authorized user can perform some distinct operations only by himself.

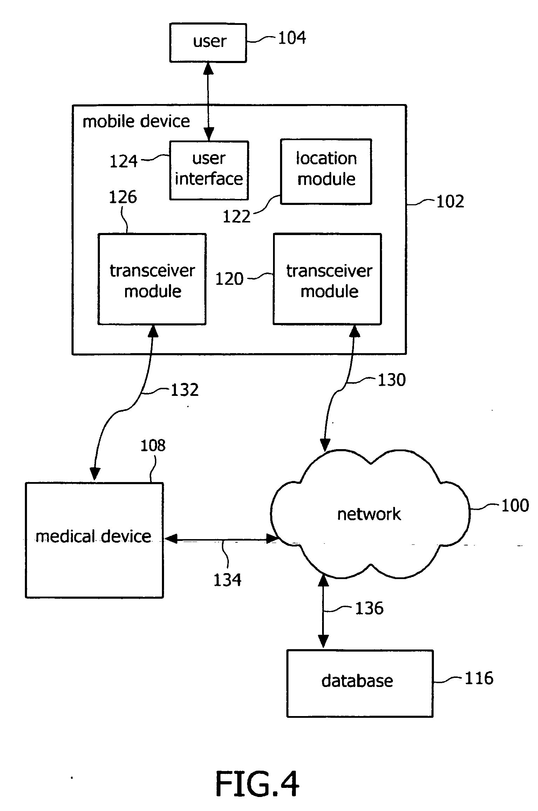

Figure 4 shows a detailed block diagram of a mobile device 102 in combination with the network 100, the network resource or medical device 108, the user 104 and the access database 116. The mobile device 102 has a user interface 124, a location module 122 and transceiver modules 120, 126. The user 104 enters a control command into the mobile device 102 by means of the user interface 124. Preferably, the user interface 124 is also adapted to provide information to a user 104. For example the user interface 124 indicates access for various functions of the network resource or medical device 108 and may further inform the user 104 of location dependent access rights. The user interface 124 may for example graphically visualize an access zone in which the mobile device is enabled to wireless entry of control commands to the medical device 108.

The location module 122 is adapted to provide location information to the mobile device 102. Therefore, the location module 122 makes for example use of a GPS unit. Alternatively, the location module 122 is adapted to transmit and receive sequences of electromagnetic signals allowing for precise determination of the location of the mobile device 102 by means of triangulation.

The transceiver module 120 is adapted to wirelessly communicate with the network via the wireless communication link 130. Wireless communication is typically realized by means of infrared or radio frequency technology and is typically based on communication standards such as Bluetooth, IrDA, IEEE 802.11 or UWB. In a similar way transceiver module 126 is adapted to wirelessly communicate with the network resource or medical device 108 via the wireless communication link 132. The network resource or medical device 108 is adapted to communicate with the network 100 via the fixed communication link 134. Additionally, network 100 is adapted for accessing the database 116 via communication link 136.

The location of the mobile device 102 can either be determined by the location module 122 in a self determined way or by means of communicating with the network 100. Therefore, the transceiver module 120 can be effectively used for transmission of control commands of the user 104 as well as for determination of the location of the mobile device 102.

Moreover, evaluation of an access condition can either be performed by the network 100, by the network resource or medical device 108 or even by the mobile device 102 itself. Transceiver module 126 is adapted to wirelessly communicate with the medical device 108 in order to determine location of the medical device 108, and/or to transmit control commands to the medical device 108 from the mobile device 102. Evaluation of an access condition by the mobile device 102 only requires that location information of the medical device 108, location information of the mobile device 102 as well as additional information from the database 116 are provided to the mobile device 102.

Predefined threshold values indicating relevant distances between the medical device 108 and the mobile device 102 are typically stored in the database 116. By means of the communication link 136 and the wireless communication link 130, the mobile device 102 is adapted to access relevant threshold parameters specified in the database 116. In this way, the entire set of parameters needed for evaluating an access condition are provided to the mobile device 102. When an access condition is positively evaluated, a control command of the user 104 can be directly submitted to the medical device 108 via the wireless communication link 132.

In a similar way also the medical device 108 might be able to evaluate an access condition of the mobile device 102 or an access condition of the user 104, respectively. By means of the communication link 134 and the wireless communication link 132, the medical device 108 is adapted to receive location information of the mobile device 102, an identification of the user 104 as well as further predefined threshold values specified by the database 116. Upon evaluation of the access condition, the medical device 108 may enable or disable a control command of the user 104 received from the transceiver module 126.

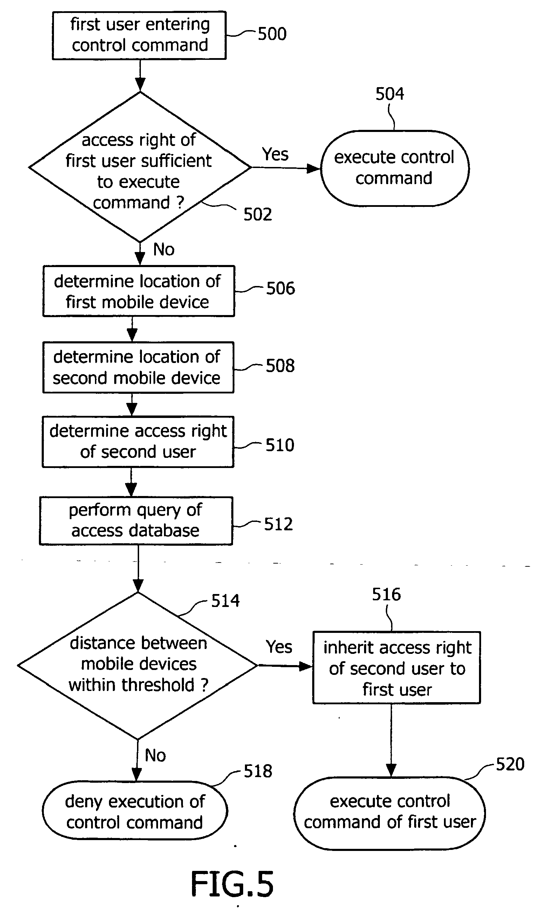

Figure 5 is illustrative of a flow chart for location dependent inheritance of access rights between a first and a second user making use of first and second mobile devices. In a first step 500 the first user enters a control command into the first mobile device. In the subsequent step 502, it is checked whether the default access rights of the first user are sufficient to execute the entered control command. If the access right of the first user is sufficient to execute the control command, the method continues with step 504. The control command is then submitted to a network resource and/or to a medical device where the control command is executed.

In the other case, when the default access right of the first user is insufficient to execute the command, after step 502 the method continues with step 506. In step 506 a first location of the first mobile device is determined and in the subsequent step 508 a second location of a second mobile device being operated by a second user is determined. After the location of the second mobile device has been determined in step 508, the default access rights of the second user making use of the second mobile device are determined in the following step 510. Hence the location of the first mobile device, the second mobile device the identity of the first and the second user making use of the first and the second mobile device have been determined respectively. Also, the corresponding default access rights of the first and the second user are known.

By making use of these parameters in the following step 512 a query of the access database is performed. In response of this query, it is checked in the subsequent step 514 whether the distance between the two mobile devices is within a predefined threshold specified by an appropriate access condition that takes access rights of the first and the second user into account. If the distance between the mobile devices is within the predefined threshold, the method continues with step 516 where access rights of the second user are inherited to the first user. After inheritance of access rights in step 516, the method continues with step 520, where the first user is enabled to execute the control command. In the opposite case, if the distance between the first and the second mobile devices is not within the predefined threshold, the method continues with step 518 where execution of the control command is denied.

### LIST OF REFERENCE NUMERALS

- 100: network
- 102: mobile device
- 104: user
- 106: user ID
- 108: medical device
- 110: mobile device
- 112: user
- 114: user ID
- 116: access database
- 120: transceiver module
- 122: location module
- 124: user interface
- 126: transceiver module
- 130: wireless link
- 132: wireless link
- 134: link
- 136: link

## Claims

1. A method of wireless entry of a control command from a first mobile device (102) into a medical device (108), the method comprising the steps of:
- determining a first location of the first mobile device,
- evaluating an access condition for the wireless entry from the first mobile device, the access condition specifying which functionality of the medical device can be accessed and depending on the distance between the first location and the location of the medical device,
- determining of a first user identity, ID, (106) of a first user (104) entering the control command into the first mobile device (102), whereby the evaluation of the access condition further depends on the first user ID,
- determining of a second user ID (114) of a second user (112) of a second mobile device (110),
- determining a second location of the second mobile device, whereby evaluating of the access condition further depends on the second user ID and the distance between the first location and the second location,
inheriting second access rights of the second user, specifying a predefined level of authorization of the second user, to first access rights of the first user, specifying a predefined level of authorization of the first user, if the distance between the first and the second location is within a predefined threshold, evaluation of the access condition further depending on the first and second access rights, - enabling the wireless entry of the control command if the access condition is fulfilled.

2. The method according to claim 1, whereby enabling of the wireless entry and evaluation of the access condition being repeatedly performed in order to respond to a modification of the distance between the first and the second location and/or a modification of the distance between the first location and the location of the medical device (108).

3. A mobile device (102, 110) for wireless entry of a control command into a medical device (108), the mobile device comprising:
- means for entering the control command into the mobile device,
- means for determining a first location of the mobile device,
- means for evaluating an access condition for the wireless entry of the control command, the access condition specifying which functionality of the medical device can be accessed and depending on the distance between the first location and a location of the medical device,
- means for determining a user identity, ID, (106) of a first user (104), the first user using the mobile device,
- means for determining a second location of a second mobile device (110),
- means for determining a second user ID (114) of a second user (116), the second user using the second mobile device (110),
whereby the access condition further depends on the first user ID, the distance between the first location and the second location and the second user ID,
- means for inheriting second access rights of the second user, specifying a predefined level of authorization of the second user, to first access rights of the first user, specifying a predefined level of authorization of the first user, if the distance between the first and the second location is within a predefined threshold, evaluation of the access condition further depending on the first and second access rights,
- means for enabling the wireless entry of the control command if the access condition is fulfilled.

4. A network element for wireless entry of a control command into a medical device, the network element comprising:
- means for wireless receiving of the control command from a first mobile device,
- means for determining a first location of the first mobile device,
- means for evaluating an access condition for the wireless entry of the control command, the access condition specifying which functionality of the medical device can be accessed and depending on the distance between the first location and a location of the medical device,
- means for determining a first user identity, ID, of a first user, the first user using the first mobile device,
- means for determining a second location of a second mobile device,
- means for determining a second user ID of a second user, the second user using the second mobile device,
whereby the access condition further depends on the first user ID, the distance between the first location and the second location and the second user ID,
- means for inheriting second access rights of the second user, specifying a predefined level of authorization of the second user, to first access rights of the first user, specifying a predefmed level of authorization of the first user, if the distance between the first and the second location is within a predefined threshold, evaluation of the access condition further depending on the first and second access rights,
- means for enabling the wireless entry of the control command if the access condition is fulfilled.

5. The network element according to claim 4, further comprising an access database, the access database being adapted to store assignments between the access rights, user IDs and first locations, the access database being further adapted to provide the access right of the user in response to receiving a first location and a first user ID.

## Patentansprüche

1. Verfahren der drahtlosen Eingabe eines Steuerbefehls von einer ersten mobilen Einrichtung (102) in eine medizinische Einrichtung (108), wobei das Verfahren die folgenden Schritte umfasst:
- Bestimmen eines ersten Standorts der ersten mobilen Einrichtung,
- Auswerten einer Zugriffsbedingung für die drahtlose Eingabe von der ersten mobilen Einrichtung aus, wobei die Zugriffsbedingung festlegt, auf welche Funktionen der medizinischen Einrichtung zugegriffen werden kann, und von dem Abstand zwischen dem ersten Standort und dem Standort der medizinischen Einrichtung abhängt,
- Bestimmen einer ersten Benutzeridentität ID (106) eines ersten Benutzers (104), der den Steuerbefehl in die erste mobile Einrichtung (102) eingibt, wobei die Auswertung der Zugriffsbedingung ferner von der ersten Benutzeridentität abhängt,
- Bestimmen einer zweiten Benutzeridentität ID (114) eines zweiten Benutzers (112) einer zweiten mobilen Einrichtung (110),
- Bestimmen eines zweiten Standorts der zweiten mobilen Einrichtung, wobei das Auswerten der Zugriffsbedingung ferner von der zweiten Benutzeridentität und dem Abstand zwischen dem ersten Standort und dem zweiten Standort abhängt,
- Übernehmen der zweiten Zugriffsrechte des zweiten Benutzers, Festlegen einer vorbestimmten Stufe der Berechtigung des zweiten Benutzers für erste Zugriffsrechte des ersten Benutzers, Festlegen einer vorbestimmten Stufe der Berechtigung des ersten Benutzers, wenn der Abstand zwischen dem ersten und dem zweiten Standort innerhalb eines vorbestimmten Schwellenwertes liegt, wobei die Auswertung der Zugriffsbedingung ferner von auf den ersten und zweiten Zugriffsrechten abhängt,
- Freigeben der drahtlosen Eingabe des Steuerbefehls, wenn die Zugriffsbedingung erfüllt ist.

2. Verfahren nach Anspruch 1, wobei das Freigeben der drahtlosen Eingabe und die Auswertung der Zugriffsbedingung wiederholt durchgeführt werden, um auf eine Änderung des Abstands zwischen dem ersten und dem zweiten Standort und/oder einer Änderung des Abstands zwischen dem ersten Standort und dem Standort der medizinischen Einrichtung (108) zu reagieren.

3. Mobile Einrichtung (102, 110) für die drahtlose Eingabe eines Steuerbefehls in eine medizinische Einrichtung (108), wobei die mobile Einrichtung Folgendes umfasst:
- Mittel zum Eingeben des Steuerbefehls in die mobile Einrichtung,
- Mittel zum Bestimmen eines ersten Standorts der mobilen Einrichtung,
- Mittel zum Auswerten einer Zugriffsbedingung für die drahtlose Eingabe des Steuerbefehls, wobei die Zugriffsbedingung festlegt, auf welche Funktionen der medizinischen Einrichtung zugegriffen werden kann, und von dem Abstand zwischen dem ersten Standort und dem Standort der medizinischen Einrichtung abhängt,
- Mittel zum Bestimmen einer ersten Benutzeridentität ID (106) eines ersten Benutzers (104), wobei der erste Benutzer die mobile Einrichtung benutzt,
- Mittel zum Bestimmen eines zweiten Standorts einer zweiten mobilen Einrichtung (110),
- Mittel zum Bestimmen einer zweiten Benutzeridentität ID (114) eines zweiten Benutzers (116), wobei der zweite Benutzer die zweite mobile Einrichtung (110) benutzt,
wobei die Zugriffsbedingung ferner von der ersten Benutzeridentität, dem Abstand zwischen dem ersten Standort und dem zweiten Standort und der zweiten Benutzeridentität abhängt,
- Mittel zum Übernehmen der zweiten Zugriffsrechte des zweiten Benutzers, Festlegen einer vorbestimmten Stufe der Berechtigung des zweiten Benutzers für erste Zugriffsrechte des ersten Benutzers, Festlegen einer vorbestimmten Stufe der Berechtigung des ersten Benutzers, wenn der Abstand zwischen dem ersten und dem zweiten Standort innerhalb eines vorbestimmten Schwellenwertes liegt, wobei die Auswertung der Zugriffsbedingung ferner von auf den ersten und zweiten Zugriffsrechten abhängt,
- Mittel zum Freigeben der drahtlosen Eingabe des Steuerbefehls, wenn die Zugriffsbedingung erfüllt ist.

4. Netzwerkbauteil für die drahtlose Eingabe eines Steuerbefehls in eine medizinische Einrichtung, wobei das Netzwerkbauteil Folgendes umfasst:
- Mittel zum drahtlosen Empfangen des Steuerbefehls von einer ersten mobilen Einrichtung,
- Mittel zum Bestimmen eines ersten Standorts der ersten mobilen Einrichtung,
- Mittel zum Auswerten einer Zugriffsbedingung für die drahtlose Eingabe des Steuerbefehls, wobei die Zugriffsbedingung festlegt, auf welchen Funktionen der medizinischen Einrichtung zugegriffen werden kann, und von dem Abstand zwischen dem ersten Standort und dem Standort der medizinischen Einrichtung abhängt,
- Mittel zum Bestimmen einer ersten Benutzeridentität ID eines ersten Benutzers, wobei der erste Benutzer die erste mobile Einrichtung benutzt,
- Mittel zum Bestimmen eines zweiten Standorts einer zweiten mobilen Einrichtung,
- Mittel zum Bestimmen einer zweiten Benutzeridentität ID eines zweiten Benutzers, wobei der zweite Benutzer die zweite mobile Einrichtung benutzt,
wobei die Zugriffsbedingung ferner von der ersten Benutzeridentität, dem Abstand zwischen dem ersten Standort und dem zweiten Standort und der zweiten Benutzeridentität abhängt,
- Mittel zum Übernehmen der zweiten Zugriffsrechte des zweiten Benutzers, Festlegen einer vorbestimmten Stufe der Berechtigung des zweiten Benutzers für erste Zugriffsrechte des ersten Benutzers, Festlegen einer vorbestimmten Stufe der Berechtigung des ersten Benutzers, wenn der Abstand zwischen dem ersten und dem zweiten Standort innerhalb eines vorbestimmten Schwellenwertes liegt, wobei die Auswertung der Zugriffsbedingung ferner von auf den ersten und zweiten Zugriffsrechten abhängt,
- Mittel zum Freigeben der drahtlosen Eingabe des Steuerbefehls, wenn die Zugriffsbedingung erfüllt ist.

5. Netzwerkbauteil nach Anspruch 4, das ferner eine Zugangsdatenbank umfasst, wobei die Zugangsdatenbank so ausgelegt ist, dass sie Zuordnungen zwischen den Zugriffsrechten, den Benutzeridentitäten und den ersten Standorten speichert, wobei die Zugangsdatenbank ferner so ausgelegt ist, dass sie das Zugriffsrecht des Benutzers als Reaktion auf dem Empfang eines ersten Standorts und einer ersten Benutzeridentität erteilt.

## Revendications

1. Procédé d'entrée sans fil d'une commande de contrôle depuis un premier dispositif mobile (102) dans un dispositif médical (108), le procédé comprenant les étapes consistant à :
- déterminer un premier emplacement du premier dispositif mobile,
- évaluer une condition d'accès pour l'entrée sans fil depuis le premier dispositif mobile, la condition d'accès spécifiant quelles fonctionnalités du dispositif médical sont accessibles et dépendant de la distance entre le premier emplacement et l'emplacement du dispositif médical,
- déterminer une identité de premier utilisateur, ID, (106) d'un premier utilisateur (104) entrant la commande de contrôle dans le premier dispositif mobile (102), de telle manière que l'évaluation de la condition d'accès dépend en outre de l'identité de premier utilisateur,
- déterminer une identité de deuxième utilisateur, ID, (114) d'un deuxième utilisateur (112) d'un deuxième dispositif mobile (110),
- déterminer un deuxième emplacement du deuxième dispositif mobile, de telle manière que l'évaluation de la condition d'accès dépend en outre de l'identité de deuxième utilisateur et de la distance entre le premier emplacement et le deuxième emplacement,
- hériter des deuxièmes droits d'accès du deuxième utilisateur, en spécifiant un niveau prédéfini d'autorisation du deuxième utilisateur, aux premiers droits d'accès du premier utilisateur, en spécifiant un niveau prédéfini d'autorisation du premier utilisateur, si la distance entre le premier emplacement et le deuxième emplacement ne dépasse pas un seuil prédéfini, l'évaluation de la condition d'accès dépendant en outre des premiers droits d'accès et des deuxièmes droits d'accès,
- permettre l'entrée sans fil de la commande de contrôle si la condition d'accès est remplie.

2. Procédé selon la revendication 1, dans lequel les étapes consistant à permettre l'entrée sans fil et à évaluer la condition d'accès sont effectuées à répétition pour répondre à une modification de la distance entre le premier emplacement et le deuxième emplacement et/ou à une modification de la distance entre le premier emplacement et l'emplacement du dispositif médical (108).

3. Dispositif mobile (102, 110) pour l'entrée sans fil d'une commande de contrôle dans un dispositif médical (108), le dispositif mobile comprenant :
- un moyen pour entrer la commande de contrôle dans le dispositif mobile,
- un moyen pour déterminer un premier emplacement du dispositif mobile,
- un moyen pour évaluer une condition d'accès pour l'entrée sans fil de la commande de contrôle, la condition d'accès spécifiant quelles fonctionnalités du dispositif médical sont accessibles et dépendant de la distance entre le premier emplacement et un emplacement du dispositif médical,
- un moyen pour déterminer une identité de premier utilisateur, ID, (106) d'un premier utilisateur (104), le premier utilisateur utilisant le dispositif mobile,
- un moyen pour déterminer un deuxième emplacement d'un deuxième dispositif mobile (110),
- un moyen pour déterminer une identité de deuxième utilisateur, ID, (114) d'un deuxième utilisateur (116), le deuxième utilisateur utilisant le deuxième dispositif mobile (110),
de telle manière que la condition d'accès dépend en outre de l'identité de premier utilisateur, de la distance entre le premier emplacement et le deuxième emplacement, et de l'identité de deuxième utilisateur,
- un moyen pour hériter des deuxièmes droits d'accès du deuxième utilisateur, en spécifiant un niveau prédéfini d'autorisation du deuxième utilisateur, aux premiers droits d'accès du premier utilisateur, en spécifiant un niveau prédéfini d'autorisation du premier utilisateur, si la distance entre le premier emplacement et le deuxième emplacement ne dépasse pas un seuil prédéfini, l'évaluation de la condition d'accès dépendant en outre des premiers droits d'accès et des deuxièmes droits d'accès,
- un moyen pour permettre l'entrée sans fil de la commande de contrôle si la condition d'accès est remplie.

4. Elément de réseau pour l'entrée sans fil d'une commande de contrôle dans un dispositif médical, l'élément de réseau comprenant :
- un moyen pour recevoir sans fil la commande de contrôle depuis un premier dispositif mobile,
- un moyen pour déterminer un premier emplacement du premier dispositif mobile,
- un moyen pour évaluer une condition d'accès pour l'entrée sans fil de la commande de contrôle, la condition d'accès spécifiant quelles fonctionnalités du dispositif médical sont accessibles et dépendant de la distance entre le premier emplacement et un emplacement du dispositif médical,
- un moyen pour déterminer une identité de premier utilisateur, ID, d'un premier utilisateur, le premier utilisateur utilisant le premier dispositif mobile,
- un moyen pour déterminer un deuxième emplacement d'un deuxième dispositif mobile,
- un moyen pour déterminer une identité de deuxième utilisateur, ID, d'un deuxième utilisateur, le deuxième utilisateur utilisant le deuxième dispositif mobile,
de telle manière que la condition d'accès dépend en outre de l'identité de premier utilisateur, de la distance entre le premier emplacement et le deuxième emplacement, et de l'identité de deuxième utilisateur,
- un moyen pour hériter des deuxièmes droits d'accès du deuxième utilisateur, en spécifiant un niveau prédéfini d'autorisation du deuxième utilisateur, aux premiers droits d'accès du premier utilisateur, en spécifiant un niveau prédéfini d'autorisation du premier utilisateur, si la distance entre le premier emplacement et le deuxième emplacement ne dépasse pas un seuil prédéfini, l'évaluation de la condition d'accès dépendant en outre des premiers droits d'accès et des deuxièmes droits d'accès,
- un moyen pour permettre l'entrée sans fil de la commande de contrôle si la condition d'accès est remplie.

5. Elément de réseau selon la revendication 4, comprenant en outre une base de données d'accès, la base de données d'accès étant apte à stocker des assignations entre les droits d'accès, les identités d'utilisateur et les premiers emplacements, la base de données d'accès étant en outre apte à fournir le droit d'accès de l'utilisateur en réponse à la réception d'un premier emplacement et d'une identité de premier utilisateur.
